(19) Europäisches Patentamt
European Patent Office
Office européen des brevets

(11) **EP 1 585 810 B1**

(12) **EUROPÄISCHE PATENTSCHRIFT**

(45) Veröffentlichungstag und Bekanntmachung des Hinweises auf die Patenterteilung:
**24.03.2010 Patentblatt 2010/12**

(21) Anmeldenummer: **03767406.6**

(22) Anmeldetag: **07.11.2003**

(51) Int Cl.:
*C12P 21/02* (2006.01)     *C12P 21/08* (2006.01)
*C12N 5/07* (2010.01)

(86) Internationale Anmeldenummer:
**PCT/DE2003/003693**

(87) Internationale Veröffentlichungsnummer:
**WO 2004/048556 (10.06.2004 Gazette 2004/24)**

(54) **VERFAHREN ZUR KULTIVIERUNG VON ZELLEN ZUR PRODUKTION VON SUBSTANZEN**

METHOD FOR CULTURING CELLS IN ORDER TO PRODUCE SUBSTANCES

PROCEDE DE MISE EN CULTURE DE CELLULES POUR PRODUIRE DES SUBSTANCES

(84) Benannte Vertragsstaaten:
**AT BE BG CH CY CZ DE DK EE ES FI FR GB GR HU IE IT LI LU MC NL PT RO SE SI SK TR**

(30) Priorität: **27.11.2002 DE 10255508**

(43) Veröffentlichungstag der Anmeldung:
**19.10.2005 Patentblatt 2005/42**

(60) Teilanmeldung:
**10152393.4**

(73) Patentinhaber: **F.HOFFMANN-LA ROCHE AG**
**4070 Basel (CH)**

(72) Erfinder:
• **LINK, Thomas**
**82377 Penzberg (DE)**
• **ESSERS, Ruth**
**52064 Aachen (DE)**
• **SKOPNIK, Kerstin**
**50169 Kerpen (DE)**
• **GÄTGENS, Jochen**
**52428 Jülich (DE)**
• **NOLL, Thomas**
**52428 Jülich (DE)**
• **WANDREY, Christian**
**52428 Jülich (DE)**

(74) Vertreter: **Schreiner, Siegfried et al**
**Roche Diagnostics GmbH**
**Patent Department (TR-E)**
**Nonnenwald 2**
**82377 Penzberg (DE)**

(56) Entgegenhaltungen:
**WO-A-93/22448     WO-A-98/41611**
**US-A- 4 657 863     US-A1- 2001 009 767**

**Beschreibung**

Die Erfindung betrifft ein Verfahren zur Kultivierung von Zellen zur Produktion von Substanzen nach dem Oberbegriff des Anspruchs 1.

Bei der Produktion von Substanzen, insbesondere Proteinen, werden Zellkulturen in fermentativen Prozessen eingesetzt. Es können dabei Prozesse unterschieden werden, bei denen die Zellkulturen genetisch unverändert sind und eigene Stoffwechselprodukte bilden und bei denen die Organismen genetisch so modifiziert sind, daß sie entweder eigene Substanzen, beispielsweise Proteine, vermehrt, oder fremde Substanzen beispielsweise Proteine produzieren. Die die Substanzen produzierenden Organismen werden dabei mit einem Nährmedium versorgt, welches das Überleben der Organismen garantiert und die Produktion der gewünschten Zielverbindung ermöglicht. Für diese Zwecke sind eine Vielzahl von Kulturmedien bekannt, die eine Fermentation ermöglichen. Einer der wichtigsten Bestandteile der Kulturmedien ist die Glukose. Nach dem Stand der Technik ist man regelmäßig bemüht in einem Fermentationsansatz eine Mindestkonzentration an Glukose aufrecht zu erhalten, um die Ausbeute an Zielverbindung zu optimieren. Die japanische Patentanmeldung 001 101 882 A offenbart ein Kultivierungsverfahren für Säugetierzellen bei dem eine Mindestkonzentration von 0,2 mmol/l an Glukose aufrechterhalten wird. Die US 544 39 68 offenbart ein Kultivierungsverfahren, bei dem eine Glukoselimitierung erfolgt. Das Verfahren führt jedoch nicht zu einer höheren spezifischen Produktionsrate der Zellen gegenüber der nicht limitierenden Fütterung.

In WO 98/41611 wird eine Methode zur Kultivierung von in Suspension wachsenden Zellen unter Glukosezugabe beschrieben, wobei die zugegebene Glukosemenge immer an den augenblicklichen Zustand der Kultur angepasst wird.

Es ist die Aufgabe der Erfindung ein Verfahren zur Kultivierung von Zellen zu schaffen mit dem die Produktivität einer einzelnen Zelle an Produkt gesteigert wird und bei dem hohe Zelldichten ermöglicht werden. Es soll eine hohe Raum-/Zeit-Ausbeute an Produkt ermöglicht werden.

Das Verfahren soll besonders einfach in der Durchführung, mit minimalem Meß-und Regelaufwand verbunden, und besonders wirtschaftlich sein.

Ausgehend vom Oberbegriff des Anspruchs 1 wird die Aufgabe überraschenderweise dadurch gelöst, daß die Kultivierung einer, Substanzen produzierende, Zellinie unter Zufuhr eines Nährmediums in der Weise erfolgt, daß sich in der Kulturlösung eine Glukoselimitierung einstellt. Der Grad der Glukoselimitierung kann definiert werden, als das Verhältnis der beobachteten spezifischen Glukoseverbrauchsrate zur maximal für diese Zellen bekannte spezifische Glukoseverbrauchsrate. Der Grad der Glukoselimitierung $DGL = qGlc/qGlc_{max}$ ($qGlc$ = momentane beobachtete, spezifische Glukoseverbrauchsrate; $qGlc_{max}$ = maximal für diese Zellen bekannten spezifischen Glukoseverbrauchsrate). DGL liegt in den Grenzen zwischen $DGL_{Erhaltung}$ und 1, wobei $DGL_{Erhaltung}$ völlige Wachstumslimitierung bedeutet und 1 bedeutet keinerlei Limitierung bzw. völliger Glukoseüberschuß.

Zusammen mit der Glukoselimitierung erfolgt ein kontinuierlicher Rückgang der Restglukosekonzentration auf eine stationäre Konzentration in der Kulturlösung, die größer 0 mmol/l, jedoch kleiner als 1 mmol/l, bevorzugt kleiner als 0,5 mmol/l ist. Es ist zu beobachten, daß mit Sinken des DGL ein weiterer Anstieg der Lebendzelldichte im Kulturgefäß erfolgen kann. Mit zunehmender Glukoselimitierung konvergiert die Zelldichte dann gegen einen Maximalwert. Daraus resultiert, daß der Grad der Glukoselimitierung gegen einen minimalen Wert konvergiert, dabei ist der DGL erfindungsgemäß größer oder gleich dem DGL, welcher zur Erhaltung der Zelle führt, (maintenance Stoffwechsel) $DGL_{Erhaltung} = qGlC_{Erhaltung}/qGlc_{max}$ ($qGlc_{Erhaltung}$ = bei reinem Erhaltungsstoffwechsel beobachtete spezifische Glukoseverbrauchsrate; $qGlc_{max}$ = maximal für diese Zellen bekannten spezifischen Glukoseverbrauchsrate), und kleiner 0,5, vorzugsweise kleiner 0,4, besonders bevorzugt kleiner 0,3.

Charakteristisch ist jedoch, daß mit der Abnahme der Glukosekonzentration keine Abnahme der Zellkonzentration in der Lösung erfolgt. Mit zunehmender Glukoselimitierung, also bei sinkendem DGL-Wert, erhöht sich die spezifische Produktivität einer Zelle. Da die Lebendzelldichte im Kulturgefäß nicht absinkt, führt dies zu einer Erhöhung der Raum-Zeit Ausbeute. Mit Eintreten der Glukoselimitierung geht phänomenologisch eine Erniedrigung der spezifischen Laktatbildungsrate einher. Die Laktatbildungsrate konvergiert gegen einen Minimalwert. Dies führt dazu, daß die Restlaktatkonzentration im Kulturgefäß absinkt, maximal gegen 0 geht. Mit der Glukoselimitierung kommt es also zu einer Umstellung des Zellmetabolismus.

Wichtig ist dabei, daß es vor Einsetzen der Glukoselimitierung zu keiner weiteren Limitierung durch andere Substrate kommt. Daher muß das Wachstumsmedium so beschaffen sein, daß Glukose zuerst limitiert ist.

Mit dem erfindungsgemäßen Verfahren wird die Raum-/Zeit-Ausbeute bei gegebener Zelldichte erhöht. Durch das erfindungsgemäße Verfahren wird die pro Zelle zur Verfügung stehende Menge an Glukose derart vermindert, daß die Glukose überwiegend in den Erhaltungsstoffwechsel und verbunden damit das Produkt und weniger in Zellwachstum eingeht. Das erfindungsgemäße Verfahren bedarf dabei nicht einer Regelung der Glukosezufütterung, so daß das Verfahren besonders einfach ist, da auf eine aufwendige Glukoseregelung verzichtet werden kann. Dadurch, daß ein geringer Medienzufluß nötig ist, werden Kosten für Glukose gespart, da weniger Glukose benötigt wird. Zudem wird eine sehr hohe Produktkonzentration erreicht. Dies kann zur Senkung der Aufarbeitungskosten führen. Das erfindungsgemäße Verfahren ermöglicht insbesondere die Steigerung der Produktion von Proteinen, ohne daß eine Zellinie für die Umsetzung des erfindungsgemäßen Verfahrens zusätzlich genetisch verändert werden muß. Die Erhöhung des Produkttiters ermöglicht die Produktion einer gewünschten Menge an Produkten in einem kleineren Kultivierungsvolumen, was zu geringeren Investitionskosten führt.

Das erfindungsgemäße Verfahren kann mit folgenden Verfahrensschritten durchgeführt werden:

Die Zellen sollen vorzugsweise in kontinuierlicher Verfahrensweise mit Zellrückhaltung, z. B. Spinfilter (Perfusionskultur) kultiviert werden. Dabei sind alle gängigen Arten von Kulturgefäßen, wie zum Beispiel Rührkessel, und Zellrückhaltemechanismen, wie beispielsweise Spinfilter, Ultraschall oder Settler geeignet. Vorzugsweise sollte das Kultursystem hohe Zelldichten ermöglichen. Vorzugsweise wird eine Zellrückhaltung realisiert, damit die Zelldichte bei Auftreten der Glukoselimitierung nicht absinken kann. Dies führt dazu, daß bei steigender Lebendzelldichte und gleichbleibender Glukosezufütterung, der DGL weiter verringert wird. Die hohe Zelldichte ermöglicht ein Absinken des DGL unter einen Wert von 0,4 bei einer eingestellten Durchflußrate in einer Größenordnung der maximalen Wachstumsrate. So können beispielhaft Durchflußraten von 0,03 - 0,05 $h^{-1}$ für die verwendete CHO MUC2-GFP-C-term Zelle, sowie die verwendete CHO/MUC1-IgG2a PH3744/25 Zelle angewendet werden.

Um eine Verringerung des DGL zu erreichen, kann die Fütterungsstrategie mit Glukose demnach wie folgt erfolgen: Die Menge an zugefütterter Glukose wird nicht mit zunehmender Lebendzelldichte erhöht, um eine Glukoselimitierung zu vermeiden. Vielmehr wird die Menge an zugefütterter Glukose während des Prozesses von Beginn an konstant gehalten. Dabei sollte die Menge an zugefütterter Glukose so gewählt sein, daß der DGL die erforderlichen Werte unterschreitet, nämlich DGL kleiner ≤ 0,5, vorzugsweise ≤ 0,4, besonders bevorzugt ≤ 0,3. Daraus resultiert, daß die Menge zugefütterter Glukose vorzugsweise nicht größer als 50 %, besonders bevorzugt nicht größer als 35 % dessen ist, was die im System bei herkömmlicher, nicht glukoselimitierter Prozeßführung zu erwartende Lebendzellzahl maximal verbrauchen kann. Nach Umstellung des Zellmetabolismus (Laktatstoffwechsel und Produktivität) kann die Menge zugefütterter Glukose langsam erhöht werden, sollte dabei jedoch nicht einen DGL von größer als 0,5, vorzugsweise größer als 0,4 ermöglichen. Dies führt zu einer weiteren Erhöhung der Lebendzelldichte bei gleichbleibend hoher Produktivität und damit erhöhter Raum-/Zeit-Ausbeute. Die Menge zugeführter Glukose kann in einem kontinuierlichen Prozeß durch die Medienzuflußrate und die Glukosekonzentration im Zufütterungsmedium beeinflußt werden. Maßgeblich ist, daß der Massenfluß an zugeführter Glukose während des Prozesses nicht oder nur in solchem Maße erhöht wird, daß der DGL einen Wert von kleiner 0,5, vorzugsweise kleiner 0,4 erreicht oder unterschreitet und dieser dann nicht mehr überschritten wird.

Vorteilhafte Weiterbildungen der Erfindung sind in den Unteransprüchen angegeben.

Im Folgenden soll die Erfindung in ihren Einzelheiten dargestellt werden.

Die Figuren zeigen beispielhafte Versuchsergebnisse.

Es zeigt:

Fig.1: Zunahme der vitalen Zellzahl [$ml^{-1}$] und Darstellung der Mediendurchflußrate [$h^{-1}$] gegen die Prozeßzeit [h] für die Produktion von MUC1-IgG2a aus CHO MUC1/IgG2a PH3744/25 Zellen im Perfu- sionsreaktor.

Fig.2: Spezifische Produktivität an MUC1- IgG2a [μg/h*E9 Zellen)] und DGL gegen die Prozeßzeit im Perfusionsreaktor.

Fig.3: Zunahme der vitalen Zellzahl [$ml^{-1}$] und mM Restglukose, aufgetragen gegen die Prozeßzeit [h] für die Produktion von MUC1-IgG2a aus CHO MUC1/IgG2a PH3744/25 Zellen im Perfusionsreaktor.

Fig.4: Glukose- und Laktatkonzentration sowie Konzentration von Glukose im Medienzu- lauf [mmol/l], aufgetragen gegen die Prozeßzeit [h] für die Produktion von MUC1-IgG2a aus CHO MUCl/IgG2a PH3744/25 Zellen im Perfusionsreaktor.

Fig.5: Zunahme der Konzentration an MUC1- IgG2a [μg/ml] und qMUC1-IgG2a [μg/h*E9 Zellen)] gegen die Zeit [h] für die Produktion von MUC1-IgG2a aus CHO MUC1/IgG2a PH3744/25 Zellen im Perfusionsreaktor.

Fig.6: Zunahme der vitalen Zellzahl [$ml^{-1}$] und Darstellung der Mediendurchflußrate [$h^{-1}$] gegen die Prozeßzeit [h] für die Produktion von MUC2-GFP-C-term aus CHO MUC2-GFP-C-term Zellen im Perfusions- reaktor.

Fig.7: Spezifische Produktivität an MUC2-GFP-C-term [nmol/h*E9 Zellen)] und DGL gegen die Prozeßzeit im Perfusionsreaktor.

Fig.8: Zunahme der vitalen Zellzahl [$ml^{-1}$] und Restglukose [mM], aufgetragen gegen die Prozeßzeit [h] für die Produktion von MUC2-GFP-C-term aus CHO MUC2-GFP-C- term Zellen im Perfusionsreaktor

Fig.9:    Glukose- und Laktatkonzentration sowie Konzentration von Glukose im Medienzu- lauf [mmol/l], aufgetragen gegen die Prozeßzeit [h] für die Produktion von MUC2-GFP-C-term aus CHO MUC2-GFP-C- term Zellen im Perfusionsreaktor

Fig.10    Zunahme der Konzentration an MUC2- GFP-C-term [nM] und qMUC2-GFP-C-term [nmol/h*E9 Zellen)] gegen die Zeit [h] für die Produktion von MUC2-GFP-C-term aus CHO MUC2-GFP-C- term Zellen im Perfusionsreaktor.

Weiterhin zeigt Tabelle 1 die Versuchsdaten aus der Anwendung des erfindungsgemäßen Verfahrens mit der CHO MUC1/IgG2a PH 3744/25 Zelle.

In Tabelle 2 sind die Versuchsdaten aus der Anwendung des erfindungsgemäßen Verfahrens mit der CHO MUC2-GFP-C-term Zelle dargestellt.

Die erfindungsgemäße Verfahrensweise kann mit verschiedenen Produktionszellinien durchgeführt werden. Die Zellinien können als Wildtyp oder als genetisch modifizierte, rekombinante Zellen eingesetzt werden. Die genetische Modifikation kann beispielsweise durch Insertion von zusätzlichen Genen des gleichen Organismus oder eines anderen Organismus in die DNA, oder einen Vektor erfolgen, oder in der Verstärkung der Aktivität bzw. Expression eines Gens durch Einbringen eines wirksameren Promotors, zum Beispiel aus CMV. Die Gene können für verschiedene Proteine kodieren, beispielsweise für Proteine, wie Fusionsproteine oder für Antikörper.

Folgende Zellinien können beispielhaft genannt werden: Säugerzellen, wie CHO Zellinien, wie zum Beispiel CHO-K1, BHK, wie BHK-21, Hybridoma, NS/0, andere Myelomazellen und Insektenzellen oder andere höhere Zellen. Besonders bevorzugt ist die Verwendung von Zellen, die nicht vorzugsweise wachstumsgekoppelt produzieren.

Eine rekombinante CHO Zellinie deren Produktivität mit der erfindungsgemäßen Verfahrensweise gesteigert werden kann ist die Zellinie CHO MUC1/IgG2a, PH 3744/25, mit der das Glykoprotein MUC1-IgG2a sekretiert werden kann. Eine weitere CHO Zellinie, nämlich CHO MUC2-GFP-C-term, ist befähigt ein Fusionsprotein MUC2-GFP-C-term gesteigert zu sekretieren, wenn sie der erfindungsgemäßen Verfahrensweise unterzogen wird.

Als Kulturmedium kann prinzipiell jedes glukosehaltige Medium eingesetzt werden, welches bezüglich anderer Komponenten nicht limitierend ist. Beispielhaft kann ProCHO4-CDM genannt werden. Es können auch Medien basierend auf bekannten Rezepturen, wie zum Beispiel IMDM, DMEM oder Ham's F12 eingesetzt werden, die so auf die erfindungsgemäße Verfahrensweise optimiert wurden, daß lediglich Glukoselimitierung auftritt. Dies kann zum Beispiel dadurch erreicht werden, daß andere Komponenten im Verhältnis zur Glukose höher konzentriert werden. Generell ist es auch möglich die Glukose separat vom Medium zu zu dosieren.

Der PH Bereich liegt vorzugsweise zwischen 6,7 - 7,7, besonders bevorzugt zwischen 7 - 7,3. Jedoch sind auch andere pH-Bereiche denkbar.

Der Temperaturbereich liegt vorzugsweise zwischen 35 °C - 38,5 °C, besonders bevorzugt bei 37 °C für CHO MUC1- IgG2a. Es sind aber auch andere Temperaturbereiche denkbar, wie beispielsweise < 35 °C, bei denen es nicht zur irreversiblen Zerstörung des Produkts kommt.

Mit dem erfindungsgemäßen Kultivierungsverfahren können Substanzen, wie Glykoproteine, Fusionsproteine, Antikörper, Proteine im Allgemeinen produziert werden, von denen beispielhaft MUC1-IgG2a, MUC2-GFP-C-term, EPO, Interferone, Cytokine, Wachstumsfaktoren, Hormone, PA, Immunglobuline oder Fragmente von Immunglobulinen, genannt werden können.

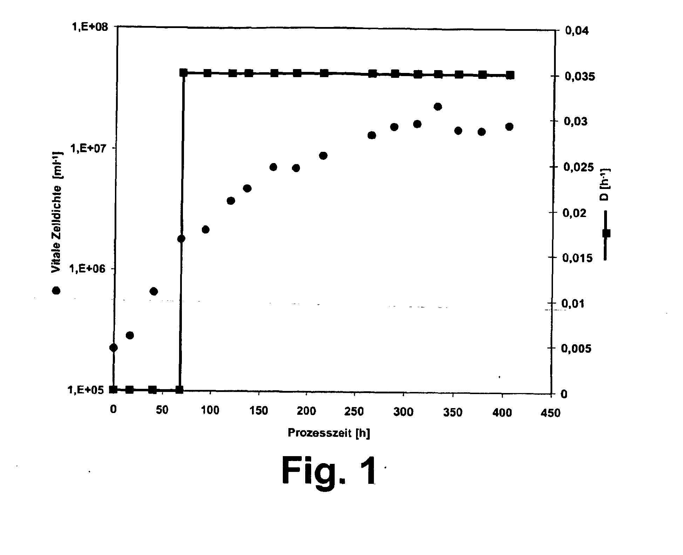

Figur 1 zeigt den Verlauf der Lebendzelldichte (cv) an CHO/MUC1-IgG2a Zellen und der Mediendurchflußrate (D) gegen die Prozeßzeit (h) im Perfusionsreaktor. In ihr ist:

■ Die Mediendurchflußrate (1/h) und
● die Lebendzelldichte (1/ml).

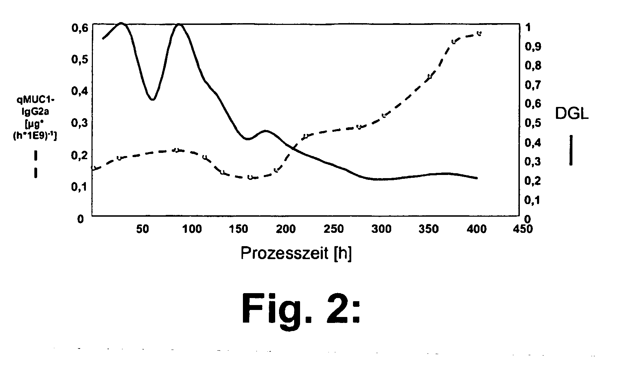

Figur 2 zeigt die spezifische Produktivität an MUC1-IgG2a (qMUC1-IgG2a) und DGL gegen die Prozeßzeit im Perfusionsreaktor. In ihr ist:

----- Die spezifische Produktivität ($\mu$g/hE9 Zellen),
—— DGL(degree of glucose limitation)

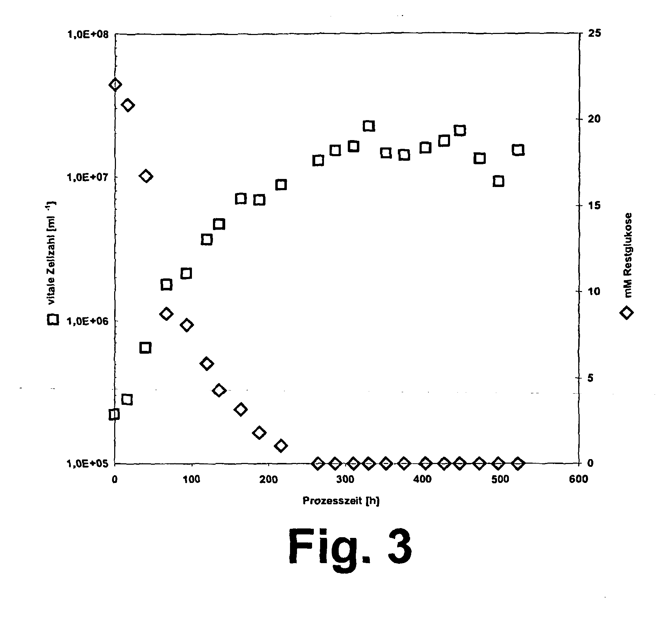

Figur 3 zeigt eine Graphik, in der auf der linken Seite die vitale Zellzahl [ml$^{-1}$] und auf der rechten Seite die Konzentration der Restglukose [mM] gegen die Prozeßzeit [h] für die Produktion von MUC1-IgG2 in CHO MUC/IgG2a PH3744/25 aufgetragen ist. In ihr ist:

□ vitale Zellzahl und
◇ Glukose.

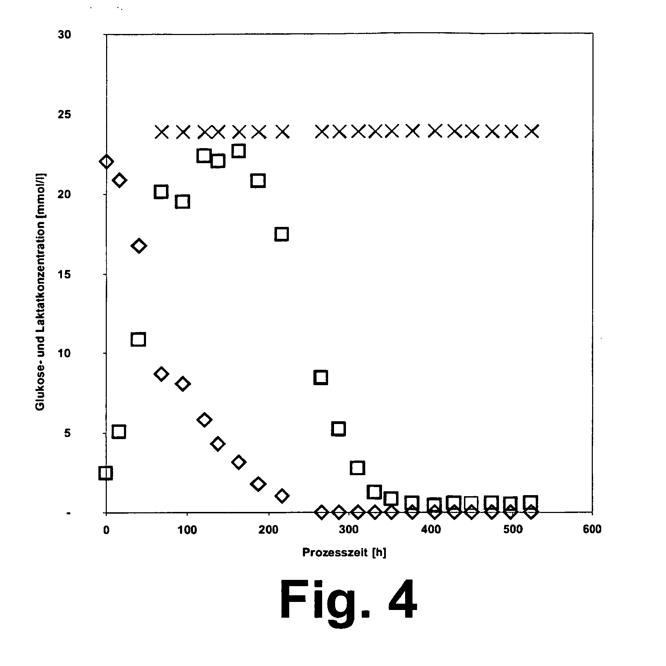

In Figur 4 ist die Glukose- und Laktatkonzentration sowie die Glukosekonzentration im Medienzulauf [mmol/l] gegen die Prozeßzeit [h] aufgetragen. In ihr sind die Kurven mit

☐ Laktatkonzentrationskurven und
◇ Glukosekonzentrationskurven.
✕ 23,9mmol/l Konzentration an Glukose im Medienzulauf (Durchflußrate von D = 0,035 h$^{-1}$).

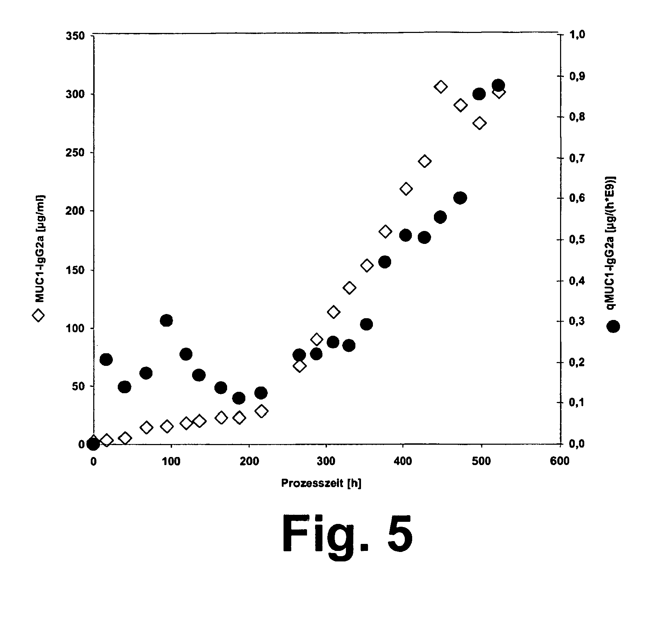

In Figur 5 ist die Konzentration von MUC1-IgG2a [μg/ml] auf der linken Seite sowie qMUC1-IgG2a [μg/(h*E9 Zellen)] auf der rechten Seite der Graphik gegen die Zeit [h] aufgetragen. In ihr sind:

● q spezifische Produktivität an MUC1-IgG2a (μg/hE9 Zellen)und
◇ Konzentration an MUC1-IgG2a (mg/l).

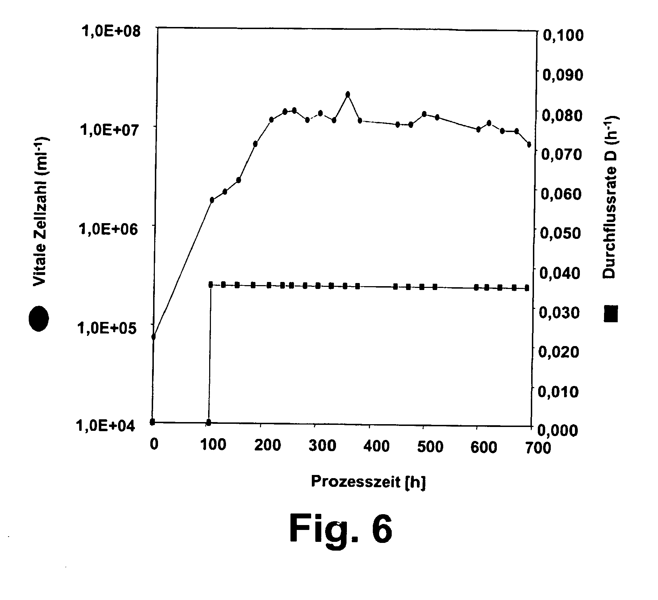

Figur 6 zeigt den Verlauf der Lebendzelldichte (cv) an CHO/MUC2-GFP Zellen und der Mediendurchflußrate (D) gegen die Prozeßzeit (h) im Perfusionsreaktor. In ihr ist:

■ Die Mediendurchflußrate (1/h) und
● die Lebendzelldichte (1/ml).

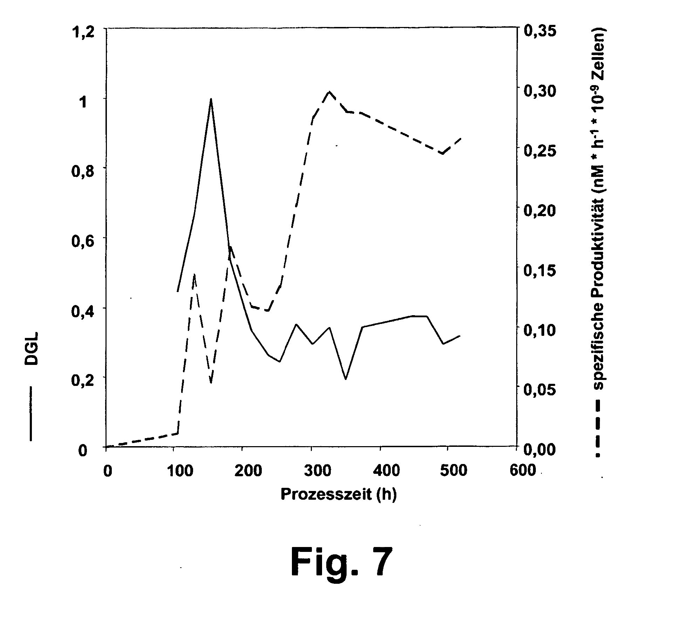

Figur 7 zeigt die spezifische Produktivität an MUC2-GFP-C-term (qMUC2-GFP-C-term) und DGL gegen die Prozeßzeit im Perfusionsreaktor. In ihr ist:

----- Die spezifische Produktivität (nmol/hE9 Zellen),
—— DGL(degree of glucose limitation)

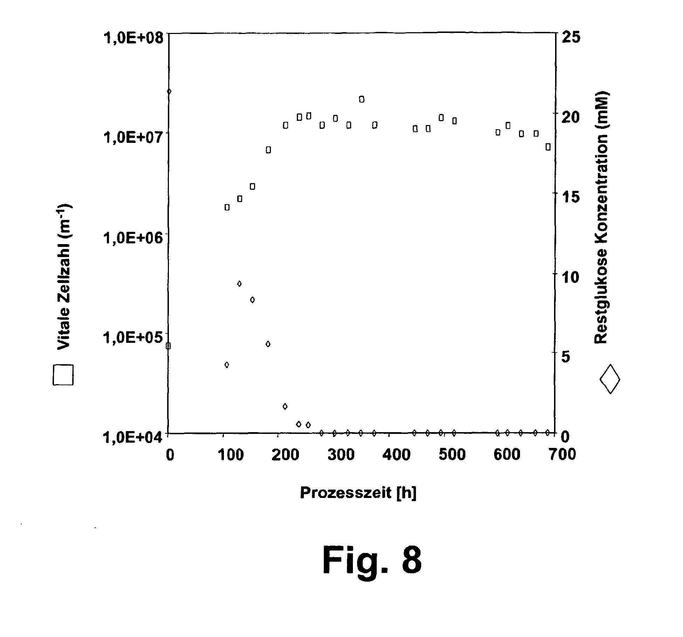

Figur 8 zeigt eine Graphik, in der auf der linken Seite die vitale Zellzahl [ml$^{-1}$] und auf der rechten Seite die Konzentration der Restglukose [mM] gegen die Prozeßzeit [h] für die Produktion von MUC2-GFP-C-term in CHO MUC/IgG2a PH3744/25 aufgetragen ist. In ihr ist:

☐ vitale Zellzahl und
◇ Glukose.

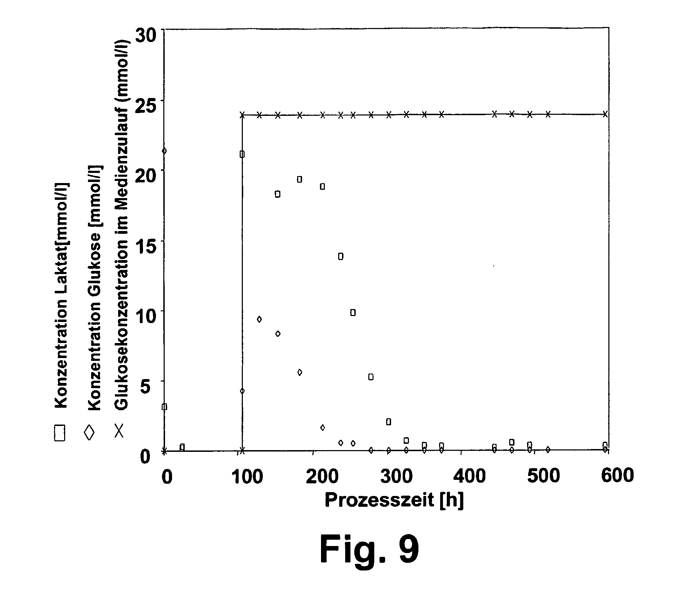

In Figur 9 ist die Glukose- und Laktatkonzentration sowie die Glukosekonzentration im Medienzulauf [mmol/l] gegen die Prozeßzeit [h] aufgetragen. In ihr sind die Kurven mit

☐ Laktatkonzentrationskurven und
◇ Glukosekonzentrationskurven.

✕ 23,9mmol/l Konzentration an Glukose im Medienzulauf (Durchflußrate von D = 0,035 h$^{-1}$).

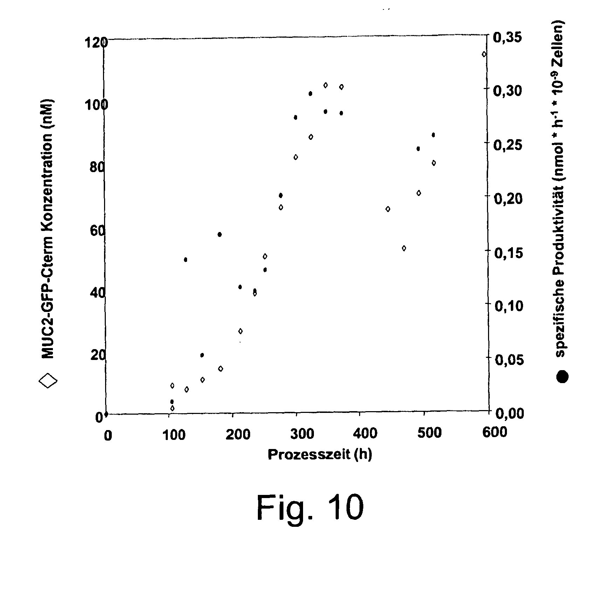

In Figur 10 ist die Konzentration von MUC2-GFP-C-term [nM] auf der linken Seite sowie qMUC2-GFP-C-term [nmol/(h*E9 Zellen)] auf der rechten Seite der Graphik gegen die Zeit [h] aufgetragen. In ihr sind:

● q spezifische Produktivität an MUC2-GFP-C-term (nmol/hE9 Zellen) und
◇ Konzentration an MUC2-GFP-C-term (nM).

Figur 1 zeigt die erfindungsgemäße Verfahrensweise beispielhaft betreffend die Glukosezufütterung. In kontinuierlicher Perfusionskultur wird eine konstante Menge Glukose zugefüttert. Im gezeigten Beispiel wird dies durch eine konstante Mediendurchflußrate erreicht, wobei die Glukosekonzentration im Medienzulauf konstant ist. Die Mediendurchflußrate wird nicht mit zunehmender Lebendzelldichte erhöht. Der Prozeß wurde als Batch begonnen, bevor die kontinuierliche Verfahrensweise begann.
Figur 2 zeigt, daß bei dieser Verfahrensweise der DGL im Verlaufe des Prozesses sinkt, und schließlich einen Wert unter 0,4 erreicht. Während dies geschieht, steigt die spezifische Produktivität an und erreicht schließlich einen Wert der um das 4-fache höher ist, als der vor Unterschreiten des DGL-Wertes von 0,4.
Aus Figur 3 wird ersichtlich, daß bei dem erfindungsgemäßen Verfahren die Lebendzelldichte gegen einen Maximalwert läuft, der dann gehalten werden kann, während die Restglukosekonzentration im Verlauf gegen null geht. Dies tritt ein, obwohl Glukose zugeführt wird. Während des Absinkens der Restglukosekonzentration, beginnt die spezifische Glukoseaufnahmerate der Organismen zu sinken. Während dessen kann die Lebendzellzahl noch ansteigen. Parallel zum Rückgang der spezifischen Glukoseaufnahmerate sinkt auch die spezifische Laktatbildungsrate, was zunächst zu einem verlangsamten Anstieg, dann zu einem Abfall der Laktatkonzentration im Kulturgefäß führt. Schließlich läuft die Laktatkonzentration im Kulturgefäß gegen null, wie aus Figur 4 zu entnehmen ist. Es liegt also eine deutliche Umstellung des Zellmetabolismus vor. Wie Figur 5 zu entnehmen ist, findet verbunden mit der Umstellung des Zellmetabolismus ein Anstieg der spezifischen Produktivität auf etwa das 4-fache gegenüber dem Zeitpunkt vor der Umstellung des Zellmetabolismus statt. Der Anstieg der spezifischen Produktivität bei mindestens gleichbleibenden, oder sogar noch ansteigenden Zelldichten während der beschriebenen Phase führt schließlich zu einem markanten Anstieg des Produkttiters im Kulturüberstand, wie Figur 5 zu entnehmen ist, und damit zu einer erhöhten Raum-/Zeit-Ausbeute.

Tabelle 1 zeigt Daten zur Fermentation von MUC1-IgG2a.

Analog zu den Figuren 1 bis 5 beschreiben Figuren 6 bis 10 die Ergebnisse der Anwendung des erfindungsgemäßen Verfahrens mit CHO MUC2-GFP-C-term Zellen.

Tabelle 2 zeigt Daten zur Fermentation von MUC2-GFP-C-term.

Produktionstechnisch kann das erfindungsgemäße Verfahren außer nach dem eben beschriebenen Perfusionsverfahren auch als Fed-Batch (Zufütterungsverfahren) betrieben werden.

In einem Fed-Batch-Betrieb wird die Produktionskultur einmal oder wiederkehrend, bzw. chargenweise oder kontinuierlich mit glukosehaltigem Medium oder einer separaten Glukoselösung in einer Art und Weise versorgt, daß der DGL vorzugsweise den Wert von 0,5, besonders bevorzugt 0,4 und noch besser 0,3 unterschreitet. Möglich ist hier auch ein repetitiver Fed-Batch.

Sowohl in der perfusiven Verfahrensweise als auch im Fed-Batch kann der Prozeß in allen allgemein bekannten Verfahrensweisen begonnen werden. So kann vor Beginn der erfindungsgemäßen Verfahrensweise die Kultur als Batch, Fed-Batch oder in kontinuierlicher Verfahrensweise mit oder auch ohne Zellrückhaltung betrieben werden.

**Tabelle 1: Daten zur Fermentation von MUC1-IgG2a**

| Prozesszeit | cv | D | Glukose Feed | Glukose | Laktat | MUC1-IgG2a | qMUC1-IgG2a | DGL |
|---|---|---|---|---|---|---|---|---|
| h | 1/ml | 1/h | mmol/l | mmol/l | mmol/l | $\mu$g/ml | $\mu$g/(h*E9) | |
| 0 | 2,23E+05 | 0 | 0 | 22,07 | 2,5 | 2,62 | | |
| 16,63 | 2,83E+05 | 0 | 0 | 20,89 | 5,1 | 3,59 | 0,21 | 0,92 |
| 40,52 | 6,48E+05 | 0 | 0 | 16,75 | 10,84 | 5,77 | 0,14 | 0,99 |
| 68 | 1,78E+06 | 0 | 0 | 8,74 | 20,1 | 14,21 | 0,17 | 0,61 |
| 94 | 2,14E+06 | 0,035 | 23,89 | 8,08 | 19,48 | 15,49 | 0,30 | 1,00 |
| 120 | 3,70E+06 | 0,035 | 23,89 | 5,84 | 22,35 | 18,02 | 0,22 | 0,72 |
| 136,5 | 4,68E+06 | 0,035 | 23,89 | 4,30 | 22,02 | 19,95 | 0,17 | 0,62 |
| 163,5 | 7,02E+06 | 0,035 | 23,89 | 3,17 | 22,66 | 22,67 | 0,14 | 0,40 |
| 187,5 | 6,96E+06 | 0,035 | 23,89 | 1,79 | 20,77 | 22,44 | 0,11 | 0,44 |
| 215,5 | 8,85E+06 | 0,035 | 23,89 | 1,04 | 17,46 | 28,24 | 0,13 | 0,35 |
| 264,75 | 1,30E+07 | 0,035 | 23,89 | - | 8,45 | 67,03 | 0,22 | 0,24 |
| 287 | 1,54E+07 | 0,035 | 23,89 | - | 5,25 | 89,42 | 0,22 | 0,20 |
| 310 | 1,64E+07 | 0,035 | 23,89 | - | 2,77 | 113,28 | 0,25 | 0,19 |
| 331 | 2,27E+07 | 0,035 | 23,89 | - | 1,24 | 133,80 | 0,24 | 0,14 |
| 352,4 | 1,45E+07 | 0,035 | 23,89 | - | 0,82 | 152,87 | 0,29 | 0,21 |
| 376,3 | 1,42E+07 | 0,035 | 23,89 | - | 0,53 | 182,52 | 0,45 | 0,22 |
| 404,4 | 1,58E+07 | 0,035 | 23,89 | - | 0,44 | 218,51 | 0,51 | 0,20 |
| 428 | 1,78E+07 | 0,035 | 23,89 | - | 0,58 | 241,75 | 0,50 | 0,17 |
| 448,4 | 2,08E+07 | 0,035 | 23,89 | - | 0,55 | 305,39 | 0,55 | 0,15 |
| 473,63 | 1,35E+07 | 0,035 | 23,89 | - | 0,55 | 290,52 | 0,60 | 0,23 |
| 496,8 | 9,30E+06 | 0,035 | 23,89 | - | 0,51 | 274,94 | 0,85 | 0,33 |
| 521,82 | 1,53E+07 | 0,035 | 23,89 | - | 0,56 | 301,12 | 0,87 | 0,20 |

**Tabelle 2:** Daten zur Fermentation von MUC2-GFP-Cterm

| Prozeßzeit h | Vitale Zellzahl 1/ml | D 1/h | Glukose Feed mmol/l | Glukose mmol/l | Laktat mmol/l | MUC2-GFP -Cterm nM | qProdukt nmol/(h*E9) | DGL |
|---|---|---|---|---|---|---|---|---|
| 0,5 | 7,50E+04 | 0 | 0 | 21,37 | 3,12 | 0,00 | | |
| 106 | 1,80E+06 | 0 | 0 | 4,25 | 21,1 | 1,66 | 0,01 | 0,44 |
| 106,01 | | 0,035 | 23,89 | | | 8,92 | | |
| 130 | 2,20E+06 | 0,035 | 23,89 | 9,36 | | 7,71 | 0,14 | 0,66 |
| 154 | 2,90E+06 | 0,035 | 23,89 | 8,32 | 18,23 | 10,72 | 0,05 | 1,00 |
| 182,38 | 6,83E+06 | 0,035 | 23,89 | 5,58 | 19,28 | 14,08 | 0,17 | 0,53 |
| 212,9 | 1,19E+07 | 0,035 | 23,89 | 1,65 | 18,78 | 26,15 | 0,12 | 0,33 |
| 237,2 | 1,44E+07 | 0,035 | 23,89 | 0,54 | 13,84 | 38,37 | 0,11 | 0,26 |
| 254 | 1,48E+07 | 0,035 | 23,89 | 0,52 | 9,81 | 50,08 | 0,13 | 0,24 |
| 278 | 1,20E+07 | 0,035 | 23,89 | - | 5,19 | 65,63 | 0,20 | 0,35 |
| 302 | 1,40 E+07 | 0,035 | 23,89 | - | 2,05 | 81,53 | 0,27 | 0,29 |
| 326 | 1,20E+07 | 0,035 | 23,89 | - | 0,7 | 88,03 | 0,30 | 0,34 |
| 349,9 | 2,16E+07 | 0,035 | 23,89 | - | 0,33 | 104,60 | 0,28 | 0,19 |
| 374 | 1,20E+07 | 0,035 | 23,89 | - | 0,26 | 104,03 | 0,28 | 0,34 |
| | | 0,035 | 23,89 | - | | 84,47 | | |
| | | 0,035 | 23,89 | - | | 75,16 | | |
| 446 | 1,10E+07 | 0,035 | 23,89 | - | 0,19 | 64,81 | | 0,37 |
| 470 | 1,10E+07 | 0,035 | 23,89 | - | 0,53 | 52,36 | | 0,37 |
| 494 | 1,40E+07 | 0,035 | 23,89 | - | 0,32 | 69,63 | 0,24 | 0,29 |
| 518 | 1,30E+07 | 0,035 | 23,89 | - | | 79,34 | 0,26 | 0,32 |
| | | 0,035 | 23,89 | - | | 93,94 | | |
| | | 0,035 | 23,89 | - | 0,35 | 104,57 | | |
| 595,8 | 1,01 E+07 | 0,035 | 23,89 | - | 0,25 | 113,89 | | |

**Patentansprüche**

1. Verfahren zur Kultivierung von Säugerzellen zur Produktion von Substanzen, **dadurch gekennzeichnet, daß** eine Substanzen produzierende Säugerzelle unter Glukoselimitierung kultiviert wird, wobei der Grad der Glukoselimitierung, DGL,

$$DGL = qGlc/qGlc_{max}$$

mit

qGlc = beobachtete momentane spezifische Glukoseverbrauchsrate,
$qGlc_{max}$ = maximal für diese Zellen bekannten spezifischen Glukoseverbrauchsrate,

größer als der Grad der Glukoselimitierung ist, welcher zur ausschließlichen Erhaltung, $DGL_{Erhaltung}$, der Zelle führt, und $\leq 0,5$ ist, wobei der

$$DGL_{Erhaltung} = qGlc_{Erhaltung}/qGlc_{max} \text{ ist,}$$

mit

$qGlc_{Erhaltung}$ = bei reinem Erhaltungsstoffwechsel beobachtete spezifische Glukoseverbrauchsrate, und
$qGlc_{max}$ = maximal für diese Zellen bekannten spezifischen Glukoseverbrauchsrate,

wobei die Menge an zugefütterter Glukose während des Verfahrens konstant gehalten wird.

2. Verfahren nach Anspruch 1, **dadurch gekennzeichnet, daß** der DgL ≤ 0,4 oder ≤ 0,3 ist.

3. Verfahren nach Anspruch 1 oder 2, **dadurch gekennzeichnet, daß** die Menge zugefütterter Glukose nicht größer als 50 % dessen ist, was die ohne Glukoselimitierung maximal erwartete Zellzahl maximal verbrauchen kann.

4. Verfahren nach Anspruch 3, **dadurch gekennzeichnet, daß** die Menge zugefütterter Glukose nicht größer als 35 % dessen ist, was die ohne Glukoselimitierung maximal erwartete Zellzahl maximal verbrauchen kann.

5. Verfahren nach einem der Ansprüche 1 bis 4, **dadurch gekennzeichnet, daß** eine Komponente aus der Gruppe der Zellinien CHO, wie z. B CHO-KI, BHK, wie z. B BHK-21, Hybridoma, Myelomazellen, wie z. B. NS/O, andere Säugerzellen und Insektenzellen oder andere höhere Zellen eingesetzt wird.

6. Verfahren nach einem der Ansprüche 1 bis 5, **dadurch gekennzeichnet, daß** die produzierten Substanzen Proteine oder Polypeptide sind.

7. Verfahren nach Anspruch 6, **dadurch gekennzeichnet, daß** die produzierten Substanzen Fusionsproteine, das Fusionsprotein MUC1-IgG2a, das Glykoprotein MUC2-GFP-C-term, EPO, Interferone, Cytokine, Wachstumsfaktoren, Hormone, PA, Immunglobuline, Fragmente von Immunglobulinen oder andere Glykoproteine sind.

8. Verfahren nach einem der Ansprüche 1 bis 7, **dadurch gekennzeichnet, daß** ein glukosehaltiges Medium eingesetzt wird, welches bezüglich anderer Nährstoffkomponenten nicht vor Eintreten der Glukoselimitierung limitierend ist.

9. Verfahren nach Anspruch 8, **dadurch gekennzeichnet, daß** die Glukose separat von anderen Substraten gefüttert wird.

10. Verfahren nach einem der Ansprüche 1 bis 9, **dadurch gekennzeichnet, daß** es in einem pH-Bereich von 6,7 - 7,7 durchgeführt wird.

11. Verfahren nach einem der Ansprüche 1 bis 10, **dadurch gekennzeichnet, daß** es in einem Temperaturbereich - zwischen 35°C und 38,5°C durchgeführt wird.

12. Verfahren nach einem der Ansprüche 1 bis 11, **dadurch gekennzeichnet, daß** es in kontinuierlicher Verfahrensweise mit zumindest partieller Zellrückhaltung betrieben wird.

13. Verfahren nach einem der Ansprüche 1 bis 12, **dadurch gekennzeichnet, daß** es im Fed-Batch Verfahren durchgeführt wird.

14. Verfahren nach einem der Ansprüche 1 bis 13, **dadurch gekennzeichnet, daß** es als Batch gestartet und als Fed-Batch oder kontinuierlicher Prozeß fortgeführt wird.

15. Verfahren nach einem der Ansprüche 1 bis 14, **dadurch gekennzeichnet, daß** es mit nicht wachstumsgekoppelt produzierenden Zellen durchgeführt wird.

**Claims**

1. A method for culturing mammalian cells to produce substances, **characterized in that** a substances producing mammalian cell is cultured under glucose limitation, wherein the degree of glucose limitation, DGL,

$$DGL = qGlc/qGlc_{max}$$

with

qGlc = currently observed specific glucose consumption rate
$qGlc_{max}$ = specific glucose consumption rate maximally known for these cells

is higher than the degree of glucose limitation that leads to exclusive maintenance of the cell, $DGL_{maintenance}$, and is ≤ 0.5, wherein the

$$DGL_{maintenance} = qGlc_{maintenance}/qGlc_{max}$$

with

$qGlc_{maintenance}$ = specific glucose consumption rate observed at pure maintenance metabolism
$qGlc_{max}$ = specific glucose consumption rate maximally known for these cells,

wherein the amount of supplemented glucose is kept constant during the method.

2. The method according to claim 1, **characterized in that** the DGL is ≤ 0.4 or ≤ 0.3.

3. The method according to claim 1 or 2, **characterized in that** the amount of supplemented glucose is not higher than 50% of what the cell number maximally expected without glucose limitation is able to consume maximally.

4. The method according to claim 3, **characterized in that** the amount of supplemented glucose is not higher than 35% of what the cell number maximally expected without glucose limitation is able to consume maximally.

5. The method according to any one of claims 1 to 4, **characterized in that** a component from the group of the cell lines CHO, such as CHO-K1, BHK, such as BHK-21, hybridoma, myeloma cells, such as NS/O, other mammalian cells and insect cells or other higher cells is used.

6. The method according to any one of claims 1 to 5, **characterized in that** the produced substances are proteins or polypeptides.

7. The method according to claim 6, **characterized in that** the produced substances are fusion proteins, the fusion protein MUC1-IgG2a, the glycoprotein MUC2-GFP-C-term, EPO, interferons, cytokines, growth factors, hormones, PA, immunoglobulins, fragments of immunoglobulins or other glycoproteins.

8. The method according to any one of claims 1 to 7, **characterized in that** a glucose-containing medium is used, which is not limiting in terms of other nutrient components before commencing of the glucose limitation.

9. The method according to claim 8, **characterized in that** the glucose is fed separately from other substrates.

10. The method according to any one of claims 1 to 9, **characterized in that** it is carried out in a pH range of from 6.7 - 7.7.

11. The method according to any one of claims 1 to 10, **characterized in that** it is carried out in a temperature range of between 35°C and 38.5°C.

12. The method according to any one of claims 1 to 11, **characterized in that** it is conducted in a continuous procedure with at least partial cell retention.

13. The method according to any one of claims 1 to 12, **characterized in that** it is carried out according to the fed-batch method.

**14.** The method according to any one of claims 1 to 13, **characterized in that** it is started as batch and continued as fed-batch or continuous process.

**15.** The method according to any one of claims 1 to 14, **characterized in that** it is carried out with non growth-coupled producing cells.

**Revendications**

**1.** Procédé de culture de cellules de mammifères pour produire des substances, **caractérisé en ce qu'**une cellule de mammifère produisant des substances est cultivée sous limitation de glucose, sachant que le taux limite de glucose, DGL,

$$DGL = qGlc/qGlc_{max}$$

où

qGlc = vitesse de consommation de glucose spécifique observé momentanément,
qGlc$_{max}$ = vitesse maximale de consommation du glucose spécifique connue pour ces cellules,

est supérieur au taux limite de glucose DGL$_{conservation}$ qui aboutit uniquement à la conservation, des cellules, et est $\leq 0,5$, sachant que

$$DGL_{conservation} = qGlc_{conservation}/qGlc_{max},$$

où qGlc$_{conservation}$ = vitesse de consommation de glucose spécifique observée en cas de pur métabolisme conservatoire, et
qGlc$_{max}$ = vitesse maximale de consommation de glucose spécifique connue pour ces cellules,
la quantité de glucose fournie pendant le procédé étant maintenue à un niveau constant.

**2.** Procédé selon la revendication 1, **caractérisé en ce que** le DGL est $\leq 0,4$ ou $\leq 0,3$.

**3.** Procédé selon la revendication 1 ou 2, **caractérisé en ce que** la quantité de glucose fournie ne dépasse pas 50 % de ce que le nombre de cellules maximal attendu sans limitation du glucose peut consommer.

**4.** Procédé selon la revendication 3, **caractérisé en ce que** la quantité de glucose fournie ne dépasse pas 35 % de ce que le nombre de cellules maximal attendu sans limitation du glucose peut consommer au maximum.

**5.** Procédé selon l'une des revendications 1 à 4, **caractérisé en ce que** l'on utilise un composant du groupe des lignées cellulaires CHO, telles que par exemple, CHO-K1, BHK, telles que par exemple, BHK-21, d'hybridomes, des cellules de myélomes telles que par exemple, NS/O, d'autres cellules de mammifères et cellules d'insectes et autres cellules supérieures.

**6.** Procédé selon l'une des revendications 1 à 5, **caractérisé en ce que** les substances produites sont des protéines ou des polypeptides.

**7.** Procédé selon la revendication 6, **caractérisé en ce que** les substances produites sont des protéines de fusion, la protéine de fusion MUC1-IgG2a, la glycoprotéine MUC2-GFP-C-term, l'EPO, des interférons, des cytokines, des facteurs de croissance, des hormones, la PA, des immunoglobulines, des fragments d'immunoglobulines ou d'autres glycoprotéines.

**8.** Procédé selon l'une des revendications 1 à 7, **caractérisé en ce que** l'on utilise un milieu contenant du glucose qui n'est pas limitant par rapport à d'autres composants nutritifs avant l'instauration de la limitation du glucose.

**9.** Procédé selon la revendication 8, **caractérisé en ce que** le glucose est fourni séparément d'autres substrats.

**10.** Procédé selon l'une des revendications 1 à 9, **caractérisé en ce qu'**il est réalisé dans une plage de pH de 6,7 à 7,7.

**11.** Procédé selon l'une des revendications 1 à 10, **caractérisé en ce qu'**il est réalisé dans une plage de températures entre 35° C et 38,5° C.

**12.** Procédé selon l'une des revendications 1 à 11, **caractérisé en ce qu'**il est exercé de façon continue avec au moins une retenue partielle des cellules.

**13.** Procédé selon l'une des revendications 1 à 12, **caractérisé en ce qu'**il est réalisé dans un procédé de type fed-batch.

**14.** Procédé selon l'une des revendications 1 à 13, **caractérisé en ce qu'**il commence par lots et se poursuit par un procédé en écoulement discontinu ou un procédé continu.

**15.** Procédé selon l'une des revendications 1 à 14, **caractérisé en ce qu'**il est réalisé avec des cellules dont la production n'est pas couplée à la croissance.

Fig. 1

**Fig. 2:**

# Fig. 3

# Fig. 4

Fig. 5

Fig. 6

# Fig. 7

**Fig. 8**

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 001101882 A
- US 5443968 A
- WO 9841611 A